(19) **Europäisches Patentamt**
**European Patent Office**
**Office européen des brevets**

(11) **EP 1 830 896 B1**

(12) **EUROPÄISCHE PATENTSCHRIFT**

(45) Veröffentlichungstag und Bekanntmachung des Hinweises auf die Patenterteilung:
**08.10.2008 Patentblatt 2008/41**

(21) Anmeldenummer: **05823229.9**

(22) Anmeldetag: **13.12.2005**

(51) Int Cl.:
*A61L 15/44* (2006.01)     *A61L 15/22* (2006.01)
*A61L 26/00* (2006.01)     *A61L 15/00* (2006.01)
*A61F 13/02* (2006.01)     *A01N 47/44* (2006.01)
*A01N 25/24* (2006.01)

(86) Internationale Anmeldenummer:
**PCT/EP2005/013340**

(87) Internationale Veröffentlichungsnummer:
**WO 2006/066752 (29.06.2006 Gazette 2006/26)**

(54) **INFEKTIONSRESISTENTE POLYURETHANSCHÄUME, VERFAHREN ZU IHRER HERSTELLUNG UND VERWENDUNG IN ANTISEPTISCH AUSGESTATTETEN WUNDAUFLAGEN**

INFECTION-RESISTANT POLYURETHANE FOAMS, METHOD FOR PRODUCING THE SAME AND USE THEREOF IN ANTISEPTIC WOUND DRESSINGS

MOUSSES POLYURETHANE RESISTANTES A L'INFECTION, LEUR PROCEDE DE PRODUCTION ET LEUR UTILISATION DANS DES PANSEMENTS A ACTION ANTISEPTIQUE

(84) Benannte Vertragsstaaten:
**AT BE BG CH CY CZ DE DK EE ES FI FR GB GR HU IE IS IT LI LT LU LV MC NL PL PT RO SE SI SK TR**
Benannte Erstreckungsstaaten:
**BA HR YU**

(30) Priorität: **21.12.2004 DE 102004061406**

(43) Veröffentlichungstag der Anmeldung:
**12.09.2007 Patentblatt 2007/37**

(73) Patentinhaber: **Bayer Innovation Gmbh**
**51368 Leverkusen (DE)**

(72) Erfinder:
• **FUGMANN, Burkhard**
**40878 Ratingen (DE)**
• **DIETZE, Melita**
**40695 Erkrath (DE)**

(74) Vertreter: **Lütjens, Henning et al**
**Bayer Business Services GmbH**
**LP-PL**
**51368 Leverkusen (DE)**

(56) Entgegenhaltungen:
**WO-A-88/01877**          **WO-A-97/00076**
**WO-A-02/100450**         **DE-A1- 4 233 289**

• **STASHAK T S ET AL: "Update on wound dressings: Indications and best use" CLINICAL TECHNIQUES IN EQUINE PRACTICE, W.B. SAUNDERS, Bd. 3, Nr. 2, Juni 2004 (2004-06), Seiten 148-163, XP004768815 ISSN: 1534-7516**

Bemerkungen:
Die Akte enthält technische Angaben, die nach dem Eingang der Anmeldung eingereicht wurden und die nicht in dieser Patentschrift enthalten sind.

**Beschreibung**

Die vorliegende Anmeldung betrifft einen infektionsresistenten Polyurethanschaum, insbesondere einen hydrophilen Polyurethanschaum, in dem das Antiseptikum Polyhexamethylenbiguanid (PHMB) und/oder dessen Hydrochlorid, mikropartikulär und/oder homogen gelöst neben einem Superabsorber in der PU-Schaumschicht vorliegt, ein Verfahren zu dessen Herstellung und dessen Verwendung in Wundauflagen.

Im Bereich der Medizin im Allgemeinen gilt es, mit infektionsresistenten Materialien eine größtmögliche Kontrolle von Infektionen bei Kontakt von körperfremdem Material mit verletztem Gewebe oder Körperflüssigkeiten zu erhalten. Durch ihre vielseitigen Eigenschaften und flexible Formgestaltung sind polymere Werkstoffe, die infektionsresistent ausgerüstet sind, für die Fertigung medizinischer Geräte oder Hilfsmittel besonders geeignet.

US 4 479 795 A beschreibt medizinisches Gerät aus permeablen Polymeren, die freisetzbare mikrobizide Substanzen enthalten, die an die Oberfläche des Polymeren diffundieren können, insbesondere Carbonsäuren.

US 5 451 424 A beschreibt eine Methode zur Herstellung medizinischen Geräts aus einer homogenen Polymerschmelze, insbesondere Polyurethan oder Polyurethan-Siloxanblockcopolymere, und Chlorhexidin.

WO 96/22114 A beschreibt medizinisches Gerät aus Polyurethan als polymerem Material, in dem ein mikrobizides Agens, Triclosan, mit bis zu, 30 Gew.-% als Weichmacher homogen gelöst ist. Das beschriebene Polyurethan wird durch Blenden eines Polyurethanharzes mit dem Triclosan hergestellt.

In GB 2085454 A wird die Verwendung eines mikrobiziden Comonomeren bei der Herstellung eines entsprechend wirkenden Polymeren beschrieben.

Ein mikrobizides polymeres Material für die Herstellung medizinischen Geräts mit PHMB als mikrobizidem Agens, das mikropartikulär im polymeren Träger vorliegt, wird in diesem Zusammenhang nicht beschrieben.

Großflächige bzw. chronische Wunden neigen besonders leicht dazu, sich zu infizieren. Antiseptisch ausgestattete polymere Wundauflagen sollen zuverlässig und hautverträglich Infektionen unterbinden und damit nachhaltig den Heilungsprozess unterstützen.

WO 03/066116 A1 = US 2003/0149406 A1 beschreibt mehrschichtige polymere Wundauflagen aus Polyurethanschaum, bevorzugt aus HYPOL, in die therapeutisch wirkende Substanzen eingebracht werden können. Mindestens eine der Schichten ist dabei ein Hydrogel, das die Wunde bedeckt. Der Wirkstoff bzw. die Wirkstoffe sind dispers im polymeren Träger verteilt bzw. in Mizellen eingeschlossen. Die Freisetzung des Therapeutikums aus den Mizellen ist auf die Mizellen in Oberflächennähe beschränkt.

US 2004/0018227 A1 beschreibt dreischichtige Wundauflagen, die u.a. aus einem porösen Polyurethanschaum bestehen, der durch physikalische Wirkung als Schwamm in der Lage ist, Wundsekret aufzunehmen. Die Polyurethanmasse kann antibakterielle Substanzen und Wachsmmsfaktoren enthalten. Superabsorber als feuchtigkeitsbindender Bestandteil werden nicht erwähnt, ebenso werden keine Beispiele gegeben, wie antibakterielle Substanzen oder Wachstumsfaktoren in das Polyurethan eingebracht werden können.

EP 1 175 148 A1 beansprucht polymere Wundauflagen aus PU Schäumen, in deren Struktur das Antiseptikum, unter anderem auch PHMB, chemisch kovalent an die Polymerstruktur gebunden ist. Hier besteht durch die kovalente Bindung an den tragenden PU Schaum nur eine beschränkte Bioverfügbarkeit.

EP 0 106 439 A1 und GB 2 290 031 A1 beschreiben mehrschichtige polymere Wundauflagen mit einer Zwischenschicht aus Polyurethanschaum, in die therapeutisch wirkende Substanzen eingebracht werden können z. B. durch Imprägnierung, wobei nicht detailliert beschrieben ist, wie die therapeutisch wirkenden Substanzen vorliegen und in welchem Umfang sie der zu behandelnden Wunde zur Verfugung stehen.

GB 2 170 713 A1 beschreibt explizit mikroporöse PU-Schwämme als Wundauflagen, die durch Aufsaugen einer Antiseptika-Lösung imprägniert werden, hier steht das Therapeutikum nur temporär zur Verfügung bis der Wirkstoff durch z. B. Wundsekret ausgeschwemmt wird.

WO 02/100450 A1 beschreibt wirkstoflhaltige, optional auch geschäumt vorliegende Polyurethangele für die Anwendung auf der Haut und/oder Wunden, die einen homogen vorliegenden Wirkstoff für die transdermale Applikation enthalten.

DD 139942 beschreibt ein Verfahren zur Herstellung einschichtiger PU Schaumwundauflagen mit antibakterieller oder anderer therapeutischer Wirkung; es wird hier allerdings immer eine hydrophile Substanz zugesetzt, die ungleich dem Wirkstoff ist, so z. B. Kohlenhydrate in Anteilen von 1-50% oder ein anderes granulationsförderndes hydrophiles Mittel.

PHMB ist allgemein als antiseptisch wirkendes Agens in der Literatur beschrieben.

WO 99/40791 A1 beschreibt z. B. einen wasserunlöslichen, transparenten, haftenden, antimikrobiellen Film, der auf Oberflächen aufgebracht wird, und sowohl kurz- als auch langfristige desinfizierende Wirkung aufweist. Der antimikrobielle Film beinhaltet ein organisches Biguanid-Polymer sowie antimikrobielles metallisches Material.

US 5869073 A1 beschreibt eine flüssige Zusammensetzung zur Oberflächenbeschichtung. Es handelt sich um eine Lösung, Dispersion oder Suspension eines biguaniden Polymers und antimikrobiellen metallischen Materials.

Durch zusätzlichen Einsatz des bi0ziden metallischen Materials wird die Fertigung der daraus erhältlichen Wundauflagen aufwendig und teuer.

US 2004/0028722 A1 und WO 02/03899 A1 beschreiben ein Cellulose-Wound-Dressing für chronische Wunden, das antimikrobielle Zusätze enthält z. B. PHMB. Cellulose hat als Trägermaterial den Nachteil, leicht mit der Wunde zu verkleben, was den Verbandswechsel erschwert und den Heilungsprozess beeinträchtigen kann.

WO 97/05910 A1 beschreibt Cellulosematerial, das eine Mischung aus PHMB und einem anionischen Polymer wie z. B. Polyacrylsäure-Superabsorber enthält. Das beschriebene Material wird in Windeln und Damenbinden verwendet, eine Anwendung im Bereich Wundversorgung wird nicht erwähnt.

US 4643181 A beschreibt einen chirurgischen Verband, der ein Substrat enthält, das von einer antimikrobiellen Klebeschicht überzogen ist. Das antimikrobielle Agens ist PHMB in bestimmten Partikelgrößen. Das Vorliegen des PHMB in der Klebeschicht kann die Bakterienbesiedlung des übrigen Verbandsmaterials jedoch nicht ausschließen.

Ebenso beschreibt WO 88/01877 A1 ein hydrophiles PU-Wound-Dressing, das eine hydrophile Gel-Klebeschicht enthält, die auch antimikrobielle Wirkstoffe enthalten kann. Hydrogele Klebeschichten allgemein haben nur eine sehr limitierte Kapazität, Wundsekret aufzunehmen. Da häufig zu diesem Zwecke noch eine Polymerschaumschicht hinzugefügt werden muss, wird die Fertigung der Wundauflage aufwendig und teuer.

Es stellt sich damit, ausgehend vom Stand der Technik, die Aufgabe, ein geeignetes mikrobizides Polymermaterial für medizinische Anwendungen, insbesondere für die Herstellung von Wundauflagen, herzustellen, das eine optimale und lang anhaltende Bioverfügbarkeit des Antiseptikums gewährleistet, dabei überschüssiges Wundsekret aufnehmen kann, einen Luft- und Feuchtigkeitsaustausch ermöglicht und einfach und billig zu fertigen ist.

Gegenstand der Erfindung sind daher zelluläre hydrophile Polyurethangele, enthaltend PHMB bzw. HUB Hydrochlorid sowie einen Superabsorber, dadurch gekennzeichnet, dass das PHMB und/oder das PHMB Hydrochlorid mikropartikulär verteilt und/oder homogen gelöst im Polyurethangel vorliegt.

Unter zellulärem Polyurethangel wird beispielsweise ein Polyurethangelschaum verstanden, welcher geschlossen- oder offenzellig, bevorzugt offenzellig sein soll. Die Schaumporengrößen bewegen sich im allgemeinen zwischen 10 und 2000 $\mu$m, bevorzugt 50 und 500 $\mu$m.

Unter mikropartikulärer Verteilung wird erfindungsgemäß eine weitgehend gleichmäßige Verteilung von fein gemahlen oder mikronisiert im Trägermaterial dispergierter Substanz verstanden. Die Partikelgröße liegt hierbei im allgemeinen in der Größenordnung von 0,5 bis 50 $\mu$m, bevorzugt werden Teilchen von 1 bis 5 $\mu$m eingesetzt.

Die weitgehend homogene mikropartikuläre Verteilung oder Lösung des PHMB bzw. des PHMB Hydrochlorids im erfindungsgemäßen Trägermaterial in Gegenwart des Superabsorbers bewirkt eine hohe Aufnahmekapazität von Flüssigkeit, insbesondere von Wundflüssigkeit, bei gleichzeitig mikrobizider Wirkung, die sofort eintritt und/oder über einen Zeitraum von 1 bis 30 Tage, bevorzugt 2 bis 15 Tage, besonders bevorzugt 5 bis 7 Tage anhält. Die Aufnahmekapazität von Flüssigkeit liegt im allgemeinen, auch in starker Abhängigkeit von deren Zusammensetzung, im Bereich des 1 bis 15 fachen, bevorzugt 2 bis 10 fachen des Gewichts des erfindungsgemäßen Polyurethangels.

Bei der Verwendung des erfindungsgemäßen Polyurethangelschaums als Wundauflage ist kennzeichnend, dass das keimhaltige Wundsekret in die Polyurethanschaummasse aufgenommen und nicht wieder in die Wunde abgegeben wird, was eine deutliche Wirkungssteigerung bei der Keimbekämpfung gegenüber dem Stand der Technik bei überraschend niedrigen Konzentrationen des Mikrobizids im Trägermaterial mit einem breiten Keimspektrum zur Folge hat.

Die Verwendung des erfindungsgemäßen Polyurethangelschaums in einer Wundauflage hat die Vorteile, dass eine Sofortwirkung und darüber hinaus eine Langzeitwirkung über mehrere Tage die Nachbildung von Gewebe ermöglicht, ohne dass dieses durch häufigen Verbandswechsel gefährdet würde. Im Zusammenspiel mit dem idealfeuchten Milieu, das durch die Kombination der Schaumstruktur des Polyurethangelträgers mit der absorbierenden Wirkung des Superabsorbers zur Aufnahme von Wundflüssigkeit erreicht wird, bewirkt dies eine gute Heilumgebung. Durch das Festhalten und Abtöten der Keime im Polyurethanschaum wird die Wunde erfolgreich vor Reinfektion geschützt. Durch möglichen Zusatz eines oder mehrerer weiterer Wirkstoffe und/oder durch Zusatz von Enzymen, Wachstumsfaktoren oder auch Zellimplantaten aus Haut- oder Stammzellen, die optional auch in zusätzlichen Schichten einer Wundauflage enthalten sein können ist eine weitere Steigerung des Heilerfolges denkbar. Das Wohlbefinden des Patienten wird durch das hautfreundliche hydrophile elastische Polyurethanschaummaterial und/oder durch zusätzlich zur Schmerzlinderung im Trägermaterial enthaltene Lokalanästhetika gesteigert. Durch die Schaumstruktur des Wundverbandes wird eine thermische Wundisolierung erreicht.

Als polymeres Material eignen sich zum einen besonders hydrophile aus nichtaromatischen Isocyanaten aufgebaute Polyurethan-Schaumgele aus

a) 2 bis 6 Hydroxylgruppen aufweisenden Polyetherpolyolen mit OH-Zahlen von 20 bis 112 und einem Ethylenoxid (EO)-Gehalt von $\geq$ 10 Gew.-%

b) Antioxydantien

c) Katalysatoren

d) Hexamethylendiisocyanat oder einem modifizierten Hexamethylendiisocyanat,

e) antimikrobiellen Wirkstoffen

f) Superabsorber

g) Schäumungsmittel

wobei das Produkt der Funktionalitäten der polyurethanbildenden Komponenten a) und d) mindestens 5,2 ist, die Katalysatormenge c) 0,005 bis 0,5 Gew.-% bezogen auf das Polyol a) beträgt, die Menge an Antioxydantien b) mindestens 0,1 Gew.-% bezogen auf Polyol a) beträgt und ein Verhältnis von freien NCO-Gruppen der Komponente d) zu den freien OH-Gruppen der Komponente a) (Isocyanatkennzahl/100) im Bereich von 0,30 bis 0,70 gewählt wird und die Menge an Wirkstoff e) 0,0001 bis 25 Gew.-% , bevorzugt 0,001 bis 5 Gew:%, besonders bevorzugt 0,01-1 Gew.%, bezogen auf das Polyol a) beträgt. Die Isocyanatkennzahl ($K$, Verhältnis der bei der Reaktion eingesetzten freien NCO-Gruppen zu den freien OH-Gruppen x 100) der erfindungsgemäßen Polyurethan-Gelmassen liegt je nach der Funktionalität der eingesetzten. Isocyanat- ($F_I$) und Polyolkomponenten ($F_p$) im Bereich von 30 bis 70, bevorzugt im Bereich von 45 bis 60. Die für die Gelbildung erforderliche Isocyanatkennzahl kann sehr einfach nach der folgenden Formel abgeschätzt werden:

$$K \approx \frac{F_I}{F_P \cdot (F_I - 1)} \times 100$$

Je nach angestrebter Klebrigkeit und Elastizität des Gels kann die tatsächlich zu verwendende Isocyanatkennzahl um bis zu $\pm$ 20% von dem berechneten Wert abweichen. Die Menge an Superabsorber f) beträgt 0,1-50 Gew.-%, bevorzugt 2-30 Gew.%, besonders bevorzugt 25-29 Gew.-% bezogen auf die Summe der Polyurethan bildenden Komponenten a) und d).

Die erfindungsgemäßen Polyetherpolyole a) sind als solche an sich bekannt und werden z. B. durch Polymerisation von Epoxiden, wie Ethylenoxid, Propylenoxid, Butylenoxid oder Tetrahydrofuran, mit sich selbst oder durch Anlagerung dieser Epoxide, vorzugsweise von Ethylenoxid und Propylenoxid - gegebenenfalls im Gemisch untereinander oder separat nacheinander - an Starterkomponenten mit mindestens zwei reaktionsfähigen Wasserstoffatomen, wie Wasser, Ethylenglykol, Propylenglykol, Diethylenglykol, Dipropylenglykol, Glyzerin, Trimethylolpropan, Pentaerythrit, Sorbit oder Succrose, hergestellt. Vertreter der genannten, zu verwendenden höhermolekularen Polyhydroxylverbindungen sind z. B. in High Polymers, Vol. XVI, "Polyurethanes, Chemistry and Technology" (Saunders-Frisch, Interscience Publishers, New York, Bd. 1, 1962, S. 32-34) aufgeführt. Erfindungsgemäß werden bevorzugt 3 bis 4, besonders bevorzugt 4 Hydroxylgruppen aufweisende Polyetherpolyole eingesetzt mit einer OH-Zahl im Bereich von 20-112, bevorzugt 30-56. Der Ethylenoxidgehalt liegt bei den erfindungsgemäß eingesetzten Polyetherpolyolen bei vorzugsweise $\geq$ 20 Gew.-%.

Als Isocyanatkomponente d) wird monomeres oder trimerisiertes Hexamethylendiisocyanat oder durch Biuret-, Uretdion-, Allophanatgruppen oder durch Prepolymerisierung mit Polyetherpolyolen oder Mischungen von Polyetherpolyolen auf Basis der bekannten Starterkomponenten mit mindestens 2 reaktionsfähigen H-Atomen und Epoxiden, wie Ethylenoxid oder Propylenoxid einer OH-Zahl von $\leq$ 850, bevorzugt 100 bis 600, modifiziertes Hexamethylendiisocyanat zugesetzt. Bevorzugt ist der Einsatz von modifiziertem Hexamethylendiisocyanat, insbesondere durch Prepolymerisation mit Polyetherdiolen der OH-Zahl 200 bis 600, deren Restgehalt an monomerem Hexamethylendiisocyanat unter 0,5 Gew.-%-liegt.

Als Katalysatoren c) kommen für die Reaktion zwischen Hydroxyl- und Isocyanatgruppen aktive, vorzugsweise die als allgemein bekannt in der Polyurethanchemie eingesetzten in Frage. Beispielsweise verwendet man tertiäre Amine, wie Triethylamin, N-Tetramethylethylendiamin, 1,4-Diazabicyclo[2,2,2]octan, N,N-Dimethylbenzylamin, N-Methyl-N'-dimethylaminoethylpiperazin, Pentamethyldiethylentriamin, oder auch als Katalysatoren bekannte Mannich-Basen aus sekundären Aminen, wie aus Dimethylamin und Aldehyden (Formaldehyd) oder Ketonen (Aceton) und Phenolen, oder Silaamine mit C-SI-Bindungen, wie 2,2;4-Trirnethyl-2-silamorpholin und 1,3-Diethylamino-methyl-tetramethyl-disiloxan. Weiter kommen auch organische Metallverbindungen, insbesondere Zinnverbindungen, wie Zinn(II)-acetat, Zinn(II)-ethylhexoat, Zinn(IV)-verbindungen wie z. B. Dibutylzinndichlorid, Dibutylzinndilaurat, Dibutylzinnmaleat, in den wasserfreien Polyetherlyolen a) lösliche Wismut(III)-carboxylate auf Basis linearer, verzweigter, gesättigter oder ungesättigter Carbonsäuren mit 2 bis 18, vorzugsweise 6 bis 18 C-Atomen in Frage. Bevorzugt sind Bi(III)-Salze verzweigter gesättigter Carbonsäuren mit tertiären Carboxylgruppen, wie der 2,2-Dimethyloctansäure (z. B. Versatic-Säuren, Shell). Gut geeignet sind Zubereitungen dieser Bi(III)-Salze in überschüssigen Anteilen dieser Carbonsäuren. Hervorragend bewährt sich eine Lösung von 1 mol des Bi (III)-Salzes der Versatic 10-Säure (2,2-Dimethyloctansäure) in einem Überschuss von 3 mol dieser Säure mit einem Bi-Gehalt von ca. 17%. (Bi(III)-neodecanoat, Coscat 83 der Fa. Brenntag NV). Weitere geeignete Katalysatoren sind in der DE-OS 29 20 501 auf den Seiten 29, Zeile 5 bis Seite 31, Zeile 25 beschrieben.

Es werden die Katalysatoren bevorzugt in Mengen von 0,03 bis 0,2 Gew.-%, bezogen auf das Polyol a), eingesetzt.

Als Antioxidantien b) kommen für die erfindungsgemäßen Poylurethangele insbesondere sterisch gehinderte phenolische Stabilisatoren, wie BHT (2,6-Di-tert.-butyl-4-methylphenol), Vulkanox BKF (2,2'-Methylen-bis(6-tert.-butyl-4-methylphenol) (Lanxess), Irganox 1010 (Pentaerythrityltetrakis-[3-(3,5-ditert.-butyl-4-hydroxyphenyl)propionat]), Irganox 1076 (Octadecyl-3-(3,5- ditert.-butyl-4-hydroxyphenyl)propionat) (Ciba Specialty Chemicals) oder Tocopherol (Vitamin E) in betracht. Bevorzugt werden solche vom Typ des $\alpha$-Tocopherol eingesetzt. Weitere Stabilisatoren sind beispielsweise in Ullmann (5. Auflage, Vol. A3, S. 91-111; Vol. A20, S. 461-479; Vol. A23, S. 381-391) genannt. Die Stabilisierungseigenschaften der phenolischen Stabilisatoren können durch Zusatz von organisch substituierten Sulfiden oder Disulfiden, wie z. B. Irganox PS800 (3,3'-Thiopropionsäuredilaurylester) oder Dioctyldidecyldisulfid, noch verbessert werden. Auch Kombinationen der phenolischen Typen untereinander sind möglich.

Die Antioxydantien werden bevorzugt in Mengen von 0,1 bis 2,0 Gew.-%, insbesondere 0,15 bis 0,5 Gew.-%, bezogen auf das Polyol a), eingesetzt. Bei Antioxydationsgemischen, wie sie oben erwähnt sind, werden die Antioxydantien bevorzugt in Mengen von 0,05 bis 0,5 Gew.-% je Einzelsubstanz, bezogen auf das Polyol a), eingesetzt.

Als Wirkstoffe e) werden für die erfindungsgemäßen Polyurethan-Gelschäume mindestens ein Antiseptikum, dabei bevorzugt Polyhexamethylenbiguanid (PHMB) oder seine Salze, besonders bevorzugt das Hydrochlorid des PHMB, aber auch zusätzlich andere Wirkstoffe aus der Gruppe der Breitbandantibiotika, der antiviralen Wirkstoffe, der antifungischen Wirkstoffe, der antipathogenen Peptide, der lokalen Anästethika, der antiseptisch, hämostatisch, wundheilend, immunmodulatorisch, granulationsfördernd oder auf das zentrale Nervensystem wirkenden Wirkstoffe, der Enzyme, vor allem solcher mit antibakterieller Wirkung, z. B. Lysozym, Papain, Trypsin, Bactilysin, Glucoseoxidase), der Wachstumsfaktoren, vor allem epidermal growth factor (EGF), platelet derived growth factor (PDGF), transforming growth factor alpha/beta (TGF), insulin-like growth factor (IGF, ILGF), fibroblast growth factor (FGF), vascular endothelial growth factor (VEGF), blood-derived growth factor (BDGF), tissue growth factor oder growth- and Amelogenin-like factors (GAF) oder Zellimplantate aus Haut- oder Stammzellen einzeln oder in Kombination eingesetzt.

Aus der Gruppe der Breitbandantibiotika eignen sich insbesondere Fosfomycin, Gentamicin oder Mupirocin sowie antibakterielle Chinoloncarbonsäuren, aus der Gruppe der antiviralen Wirkstoffe die der nukleosidanalogen Virustatika wie Aciclovir, Ganciclovir, Vidarabin, Zidovudin oder auch Foscarnet, unter den antifungischen Wirkstoffen insbesondere die Azol-Antimykotika wie Fluconazol, Clotrimazol oder Itraconazol und die Allylamine wie Terbinafin oder Morpholine wie Amorolfin oder Polyene wie Natamycin, unter den antipathogenen Peptiden z. B. Lysozym, den lokalen Anästhetika z. B. Lidocain, Benzocain, Bupivacain oder Procain. Ganz besonders bevorzugt setzt man Polyhexamethylenbiguanid Hydrochlorid alleine ein.

Superabsorber f) sind bekannte Wasser absorbierende Salze von Polyacrylaten und deren Copolymeren, insbesondere die Natrium- und Kaliumsalze. Sie können unvernetzt oder vernetzt sein und sind auch als Handelsprodukte erhältlich. Insbesondere sind solche Produkte geeignet, wie sie in der DE 37 13 601 A1 offenbart werden, und auch Superabsorber der neuen Generation mit nur noch geringen Anteilen an austrocknenbarem Wasser und hohem Quellvermögen unter Druck. Besonders bevorzugte Produkte sind schwach vernetzte Polymerisate auf der Basis von Acrylsäure/Natriumacrylat. Diese sind als Favor T (Degussa AG) erhältlich. Weitere Absorber z. B. Carboxymethylcellulose und Karaya, sind ebenfalls geeignet.

Als Schäumungsmittel g) können in der Polyurethanchemie gängige Schäumungsmittel eingesetzt werden. Hierzu zählt z. B. chemisch in situ erzeugtes Kohlendioxid als Reaktionsprodukt aus Isocyanat mit Wasser oder physikalisch wirkende, wasserfreie Treibmittel wie niedrig siedende Flüssigkeiten, beispielsweise FKW 113, HFCKW 22 oder n- und iso-Pentan, Cyclopentan, Butane und Hexane. Weitere sind beispielsweise in Becker/Braun, Kunststoff-Handbuch, Bd. 7, Polyurethane, 3. Aufl., Carl Hanser Verlag München-Wien, 1993, S. 115-118 beschrieben. Insbesondere eignen sich jedoch inerte Gase zur Schäumung der erfindungsgemäßen Polyurethane. Hierbei werden Gase, wie z. B. Stickstoff, Edelgase oder Kohlendioxid, ohne Zusatz von Wasser mit Hilfe handelsüblicher Polyurethan-Mischtechnik eingetragen.

Der Schäumungsgrad lässt sich durch die eingearbeiteten Mengen an Schäumungsmittel in weiten Grenzen variieren.

Die erfindungsgemäßen Polyurethan-Gelmassen werden hergestellt nach üblichen Verfahren, wie sie beispielsweise beschrieben sind in Becker/Braun, Kunststoff-Handbuch, Bd. 7, Polyurethane, 3. Aufl., Carl-Hauser Verlag München Wien, 1993 S. 139 ff. Zur Durchführung der Reaktion kann man wie auch dort beschrieben unterschiedlich vorgehen. Vorzugsweise nimmt man 1-10 % der Gesamtmenge des Polyols a) welches Antioxidantien b) enthält und löst oder verteilt darin den Katalysator, welches hier als Mischung A) bezeichnet wird. Die Restmenge des Polyols a) mischt man mit den Komponenten Wirkstoff(e) e) und Superabsorber f) zur Mischung B). Die Reaktion kann man jetzt durchführen, indem man zunächst die Mischungen A) und B) mischt und darauf die Isocyanatmischung; hier das gegebenenfalls modifizierte Hexamethylendiisocyanat, d) zusetzt. Dabei werden die erfindungsgemäßen hydrophilen Polyurethangele bei der Reaktion durch unmittelbaren Zusatz des Schäumungsmittels g) als Gas oder Flüssigkeit geschäumt, wobei die Dichte des geschäumten Gels auf bis zu 1/6 der Ausgangsdichte der Gelmasse reduziert wird und das Volumen sich entsprechend vergrößert. Man kann auch alle Bestandteile A), B), d) und g) gleichzeitig zusammengeben. Verwendet man niedrig siedende Flüssigkeiten mischt man diese vorzugsweise mit einer der Komponenten A), B) oder d). Eine sinngemäß übertragbare Beschreibung findet sich in der WO 94/07935 A1 Seite 19 Zeile 9 bis Seite 22 Zeile 18.

Weiterhin kommen vorzugsweise hydrophile Polyurethangelschäume zum Einsatz, welche aus einem Polyurethangel bestehen, welches selbstklebend oder alternativ nichthaftend elastisch ist und welches

(A) 25-62 oder alternativ 15-62 Gew.-%, vorzugsweise 30-60 oder alternativ 20-57 Gew.-%, besonders bevorzugt 40-57 oder alternativ 25-47 Gew.-%, bezogen auf die Summe aus (A) und (B), eines kovalent vernetzten Polyurethans als hochmolekulare Matrix und

(B) 75-38 oder alternativ 85-38 Gew.-%, vorzugsweise 70-40 oder alternativ 80-43 Gew.-%, besonders bevorzugt 60-43 oder alternativ 75-53 Gew.-%, bezogen auf die Summe aus (A) und (B) einer oder mehrerer in der Matrix durch Nebenvalenzkräfte fest gebundenen Polyhydroxylverbindungen mit einem mittleren Molekulargewicht zwischen 1000 und 12 000, vorzugsweise zwischen 1500 und 8000, besonders bevorzugt zwischen 2000 und 6000, und einer mittleren OH-Zahl zwischen 20 und 112, vorzugsweise zwischen 25 und 84, besonders bevorzugt zwischen 28 und 56, als flüssigem Dispersionsmittel, wobei das Dispersionsmittel im wesentlichen frei ist an Hydroxylverbindungen mit einem Molekulargewicht unter 800, vorzugsweise unter 1000, besonders bevorzugt unter 1500, sowie

(C) 0,00001 bis 25 Gew.-%, bevorzugt 0,001 bis 5 Gew.-%, besonders bevorzugt 0,001-2,5%, bezogen auf die Summe aus (A) und (B), an Wirkstoff, bevorzugt Polyhexamethylenbiguanid, besonders bevorzugt dessen Hydrochlorid, und gegebenenfalls einen oder mehrere weitere Wirkstoffe sowie

(D) 0,1 bis 50 Gew.-%, bevorzugt 2-30 Gew. %, besonders bevorzugt 25-29 Gew. % bezogen auf die Summe aus (A) und (B), an Superabsorber

(E) 0,01 bis 10 Gew.-% bezogen auf die Summe aus (A) und (B) Schäumungsmittel, sowie gegebenenfalls

(F) 0 bis 100 Gew.-%, bezogen auf die Summe aus (A) und (B), an Füll- und/oder Zusatzstoffen enthält, und welches erhältlich ist durch Umsetzung einer Mischung von

I) einem oder mehreren Polyisocyanaten,

II) einer oder mehreren Polyhydroxylverbindungen mit einem mittleren Molekulargewicht zwischen 1000 und 12 000, und einer mittleren OH-Zahl zwischen 20 und 112,

III) einem oder mehreren antimikrobiellen Wirkstoffen,

IV) einem oder mehreren Wasser absorbierenden Materialien,

V) einem bei Raumtemperatur flüssigen oder gasförmigen Schäumungsmittel, sowie gegebenenfalls

VI) Katalysatoren und Beschleunigern für die Reaktion zwischen Isocyanat- und Hydroxylgruppen, sowie gegebenenfalls

VII) aus der Polyurethanchemie an sich bekannten Füll- und Zusatzstoffen,

wobei die Mischung im Wesentlichen frei ist von Hydroxylverbindungen mit einem Molekulargewicht von unter 800, die mittlere Funktionalität der Polyisocyanate ($F_I$) vorzugsweise zwischen 2 und 4 liegt, die mittlere Funktionalität der Polyhydroxylverbindung $(F_p)$ zwischen 3 und 6 beträgt und die Isocyanatkennzahl (K) der Formel

$$K = \frac{300 \pm X}{(F_I \cdot F_P) - 1} + 7$$

gehorcht, in welcher X ≤ 120, vorzugsweise X ≤ 100, besonders bevorzugt X ≤ 90 ist und die Kennzahl *K* bei Werten zwischen 50 und 70 liegt, wobei die angegebenen Mittelwerte von Molekulargewicht und OH-Zahl als Zahlenmittel zu verstehen sind.

Die Polyurethangele können aus den an sich aus der Polyurethanchemie bekannten Ausgangsverbindungen nach an sich bekannten Verfahren hergestellt werden, wie sie zum Beispiel in DE 31 03 499 A1, DE 31 03 500 A1 und EP 0 147 588 A1 beschrieben werden. Wesentlich ist jedoch, dass bei der Auswahl der gelbildenden Komponenten zur Differenzierung der Eigenschaften der gewünschten Gele die oben definierten Bedingungen eingehalten werden, damit einmal nichthaftende elastische Gele oder alternativ selbsthaftende Gele erhalten werden.

Bevorzugte Polyhydroxylverbindungen sind Polyetherpolyole, wie sie in den oben genannten Offenlegungsschriften ausführlich genannt sind.

Als Polyisocyanatkomponenten I) sind sowohl (cyclo)aliphatische als auch aromatische Isocyanate geeignet. Bevorzugte (cyclo)aliphatische Polyisocyanate sind 1,6-Hexamethylendiisocyanat sowie dessen Biurete und Trimerisate bzw. hydrierte Diphenylmethandiisocyanat ("MDI")-Typen. Bevorzugte aromatische Polyisocyanate sind solche, die durch Destillation erhalten werden, wie MDI-Gemische aus 4,4'- und 2,4'-Isomeren oder 4,4'-MDI sowie Toluylendiisocyanat ("TDI")-Typen. Die TDI-Typen können auf Grund von Modifizierungen, wie Biuretisierung oder Trimerisierung, auch höherfunktionelle Anteile beinhalten. Die die Polyisocyanate kennzeichnenden Diisocyanate können insbesondere z. B. aus der Gruppe der unmodifizierten aromatischen oder aliphatischen Diisocyanate oder aber aus durch Prepolymerisierung mit Aminen, Polyolen oder Polyetherpolyolen gebildeten modifizierten Produkten gewählt werden.

Als Polyhydroxyverbindungen II) setzt man die weiter oben bei der Beschreibung der aus nichtaromatischen Isocyanaten aufgebauten Polyurethan-Schaumgele für a) angegebenen Stoffe ein.

Als Wirkstoff III) setzt man die weiter oben bei der Beschreibung der aus nichtaromatischen Isocyanaten aufgebauten Polyurethan-Schaumgele für e) angegebenen, bevorzugten und besonders bevorzugten Stoffe ein. Ganz besonders bevorzugt setzt man Polyhexamethylenbiguanid Hydrochlorid alleine ein.

Als Wasser absorbierende Materialien IV) setzt man bevorzugt die weiter oben bei der Beschreibung der aus nichtaromatischen Isocyanaten aufgebauten Polyurethan-Schaumgele für f) genannten und bevorzugt die dort bevorzugten Superabsorber ein.

Als Schäumungsmittel V) setzt man die weiter oben bei der Beschreibung der aus nichtaromatischen Isocyanaten aufgebauten Polyurethan-Schaumgele für g) angegebenen in der Polyurethanchemie gängigen Schäumungsmittel ein. Je nach Eigenschaften sind diese dann noch im fertigen Polyurethanschaum gelöst enthalten.

Als Katalysatoren und Beschleuniger VI) für die Reaktion zwischen Isocyanat- und Hydroxylgruppen setzt man gegebenenfalls die weiter oben bei der Beschreibung der aus nichtaromatischen Isocyanaten aufgebauten Polyurethan-Schaumgele für c) angegebenen Stoffe ein.

Als Füll- und Zusatzstoffe VII) werden aus der Polyurethanchemie an sich bekannte Zusatzstoffe verwendet. Als solche sind gegebenenfalls die weiter oben bei der Beschreibung der aus nichtaromatischen Isocyanaten aufgebauten Polyurethan-Schaumgele Antioxydantien zu verstehen. Erfindungsgemäß können den hydrophilen Schaumgelmassen Füllstoffe, Farbstoffe, Metallpigmente, Verdickungsmittel, oberflächenaktive Substanzen, Streckmittel, Harze, etc., zugesetzt werden, bevorzugt bis zu 100 Gew.-%, bezogen auf das Gesamtgewicht des Gels. Als anorganische Füllstoffe seien insbesondere genannt Pulver aus Zinkoxid, Titandioxid, Schwerspat, Kreide, Gips, Kieserit, Soda, Ceroxid, Quarzsand, Kaolin, Ruß und Mikrohohlkugeln sowie Kurzfasern, wie Glasfasern von 0,1-1 mm Länge. An organischen Füllstoffen seien insbesondere aufgeführt Pulver auf Basis von Polystyrol, Polyvinylchlorid, Harnstoff-Formaldehyd und Polyhydrazodicarbonamid, quellfähige Pulver und Fasern mit einer Faserlänge < 0,01 mm, z. B. Fasern auf Basis von Polyacrylsäuren und deren Salzen oder anderen, wie sie beispielsweise in Absorbent Polymer Technology (Brannon-Peppas, Harland, Elsevier, Amsterdam-Oxford-New York-Tokyo, 1990, S. 9-22) genannt sind, sowie als Textilfasern eingesetzte Materialien, wie z. B. Polyester- oder Polyamidfasern. Als Farbstoffe oder Farbpigmente sind insbesondere solche zu verstehen, wie sie in Lebensmitteln, Verpackungen oder Kosmetika eingesetzt werden, wie Eisenoxid- oder Chromoxidpigmente, Pigmente auf Phtalocyanin oder Monoazo-Basis. Als oberflächenaktive Substanzen seien z. B. Cellulosepulver, Aktivkohle und Kieselsäurepräparate genannt.

Zur Modifizierung der Hafteigenschaften der Gele können gegebenenfalls Zusätze von Streckmitteln und Harzen, also polymeren Vinylverbindungen, Polyacrylaten und sonstigen in der Klebstofftechnik üblichen Copolymeren bzw. auch Klebemittel auf Naturstoffbasis mit einem Gehalt von bis zu 10 Gew.-% bezogen auf die Gelmasse eingesetzt werden.

Die erfindungsgemäßen Polyurethan-Gelmassen werden hergestellt nach üblichen Verfahren, wie sie beispielsweise beschrieben sind in BeckerBraun, Kunststoff-Handbuch, Bd. 7, Polyurethane, 3. Aufl., Carl-Hauser Verlag München Wien, 1993 S. 139 ff. Zur Durchführung der Reaktion kann man wie auch dort beschrieben unterschiedlich vorgehen und unterschiedliche Maschinen einsetzen.

Die erfindungsgemäßen Polyurethanschaumgelmassen können generell verwendet werden zur Herstellung von medizinischem Gerät, insbesondere von Formkörpern und Haftschichten, bevorzugt von Produkten, die Kontakt haben mit menschlichen und tierischen Geweben, wie mit der Haut, mit Schleimhäuten, oder mit offenen Wunden oder mit Körperflüssigkeiten und Sekreten, wie z. B. Speichel, Blut, Wundflüssigkeiten, Urin, Fäkalien oder Schweiß. Die Materialien sind auch für Verklebung und Fixierung auf der Haut geeignet.

Bevorzugt ist der Einsatz der erfindungsgemäßen Polyurethanschaumgelmassen im Wundversorgungsbereich, insbesondere als schwach oder stark selbstklebende oder auch nicht haftende elastische mindestens einlagige Schicht, eingesetzt als Pflaster, Wundschnellverbände, als Wundauflage für großflächige oder chronische Wunden, z. B. Brandverletzungen, oder zur Verklebung von Wundversorgungsprodukten auf der Körperoberfläche. Sie dienen zusätzlich der Aufnahme von Blut oder Wundsekret sowie der Polsterung und thermischen Isolierung. Weitere Anwendungsgebiete z. B. sind Orthopädieartikel, Hygiene- und Kosmetikartikel oder stark Feuchte aufnehmende, quellfähige und polsternde Auflagen und Einlagen, z. B. Schuheinlagen, gegebenenfalls auch als druckverteilungsfähige Füllmassen für Kissen oder Polsterelemente.

Das erfindungsgemäße Polymer und ihre Verwendung in Wundauflagen ermöglichen z. B. folgende Vorteile:

- Bekämpfung von mikrobieller Infektion der Wunde und Verhinderung der Re-Infektion

- Aufnahme und irreversibles Festhalten des Wundsekretes und damit Unterstützung des Heilungsprozesses

- Abtöten der mit dem Wundsekret in den Schaum aufgenommenen Bakterien

- Hautverträglichkeit des Materials Polyurethan

- Das häufige Wechseln der Wundauflage (heute üblich: mehrmals täglich) wird überflüssig, ein Verbleib auf der Wunde von mindestens 3 bis zu 5 Tagen wird möglich, dadurch kann sich neues Hautgewebe besser ungestört ausbilden.

- Thermische Wundisolierung

- Es können weitere Wirkstoffe wie Antibiotika zur Unterstützung der Bakterienbekämpfung oder Lokalanästhetika zur Schmerzbekämpfung in das Polymer eingebracht und daraus langsam kontinuierlich abgegeben werden.

- Gegebenenfalls Beschleunigung der Wundheilung durch Enzyme, Wachstumsfaktoren und Zell-Implantate (e.g. Stammzellen)

Denkbare aus dem erfindungsgemäßen Polymer gefertigten Wundauflagen bestehen mindestens aus dem infektionsresistenten, ein Antiseptikum, bevorzugt PHMB, und Superabsorber enthaltenden, gegebenenfalls selbstklebenden Polyurethanschaum, der gegebenenfalls direkt mit der Wunde in Berührung kommt sowie optional einer weiteren Klebeschicht zur Fixierung der Wundauflage auf der Haut sowie optional einer luft- und nach außen feuchtigkeitsdurchlässigen Polymerfilmabdeckung aus beispielsweise Polyurethan zum mechanischen Schutz und Handhabung der Wundauflage sowie dem Schutz des erfindungsgemäßen Polyurethanschaums gegen von außen eindringende Feuchtigkeit und Mikroorganismen.

Die optionale Klebeschicht zur Fixierung des infektionsresistenten Polyurethanschaums kann ihrerseits optional mit antimikrobiellen, bevorzugt PHMB oder schmerzstillenden Substanzen ausgestattet sein. Der erfindungsgemäße Polyurethanschaum kann zur Wunde hin gegebenenfalls mit einer Schicht aus verschiedenen Materialien überzogen sein, wie z. B. Kollagen, Alginaten, Hydrokolloiden, Hydrogelen, Hydrofasern, einem Cellulose-Vlies, einer durchlässigen Silikonschicht, einem synthetischen (Polyurethan-)Polymeren oder anorganischen Kieselgelfaser-Polymeren, die ihrerseits wundheilungsfördernde, schmerzstillende, antiseptische, antibiotische Substanzen, Enzyme, Wachstumsfaktoren oder Zellen enthalten können. Die Materialien lassen Feuchtigkeit in den erfindungsgemäßen Polyurethanschaum passieren. Diese zusätzliche Schicht kann z. B. wie der Exsudat absorbierende Schaum ebenfalls PHMB enthalten, das Bakterien im direkten Wundkontakt zu bekämpfen in der Lage ist und für den beschleunigten Eintritt der durch den erfindungsgemäßen Polyurethanschaum vermittelten Wirkung sorgt. Diese zusätzliche Schicht kann als besonders hautfreundliche, atraumatische Oberfläche des erfindungsgemäßen Polyurethanschaums dienen, wenn die Wunde besonders empfindlich ist.

Die beschriebene Anordnung verschiedener Schichten in der Wundauflage kann in ein filmartiges Material steril verpackt sein, die Verpackung wird unmittelbar vor Gebrauch geöffnet.

Der Aufbau derartiger Wundauflagen ist allgemein bekannt und zum Beispiel in der WO 02/-100450 A1 beschrieben und dort in den Figuren 1 bis 6 dargestellt.

Bevorzugt, besonders bevorzugt oder ganz besonders bevorzugt sind Ausführungsformen, welche von den unter bevorzugt, besonders bevorzugt oder ganz besonders bevorzugt genannten Parametern, Verbindungen, Definitionen und Erläuterungen Gebrauch machen.

Die in der Beschreibung aufgeführten allgemeinen oder in Vorzugsbereichen aufgeführten Definitionen, Parameter, Verbindungen und Erläuterungen können jedoch auch untereinander, also zwischen den jeweiligen Bereichen und Vorzugsbereichen beliebig kombiniert werden.

Das folgende Beispiel soll die Erfindung näher erläutern. ohne sie jedoch darauf zu beschränken.

## Beispiel

### Beispiel 1

In einem Pappbecher werden 64,22 g Levagel® SN 100 [Bayer MaterialScience AG, Polyol-Mischpolymerisat aus Propylenoxid und Ethylenoxid (EO), Pentaerythrit Starter, Ethylenoxid-Endblock, OH-Zahl 4, mittleres Molgewicht 6400, EO-Gehalt 20 Gew.-%, stabilisiert mit 0,5 Gew.-% 2,6-Di-tert.-butyl-4-methylphenol (BHT)] vorgelegt, 27,52 g FAVOR®-PAC 230 (Degussa AG, Superabsorber auf Polyacrylsäure-Basis, Salz eines vernetzten gepfropften Polyacrylsäure/Polyalkohol Copolymers) und 1,00 g PHMB Hydrochlorid, welches zuvor im Vakuumtrockenschrank bei 80°C nachgetrocknet wurde, hinzugegeben und verrührt. Darauf gibt man eine unmittelbar vorher bereitete Lösung von 0,055 g Dibutylzinnlaurat in 2,70 G Levagel® SN 100 und rührt gut durch. Zuletzt gibt man 5,505 g Desmodur® E 305 (Bayer MaterialScience AG, NCO-terminiertes Prepolymer auf Basis von 7 mol Hexamethylendiisocyanat und 1 mol Polypropylenoxid von mittlerem Molgewicht von 400 g/mol, NCO-Gehalt ca. 12,3-13,3 Gew.-%) und 5 g Isopentan, rührt 1 min. durch und gießt die Masse in ein Teflonschälchen. Man erhält einen blassgelben klebrigen Schaum.

**Patentansprüche**

1. Zelluläres hydrophiles Polyurethangel, enthaltend Polyhexamethylenbiguanid, PHMB und/oder dessen Hydrochlorid als antiseptisch wirkendes Agens, **dadurch gekennzeichnet, dass** das antiseptisch wirkende Agens mikropartikulär verteilt und/oder homogen gelöst neben einem Superabsorber im Polyurethangel vorliegt.

2. Zelluläres hydrophiles Polyurethan gemäß Anspruch 1, **dadurch gekennzeichnet, dass** zusätzlich mindestens ein weiteres therapeutisch wirkendes Agens aus der Gruppe der Breitbandantibiotika, der antimikrobiellen Wirkstoffe, der antifungischen Wirkstoffe, der antipathogenen Peptide, der lokalen Anästhetika, der antiseptisch, hämostatisch, wundheilend, immunmodulatorisch, granulationsfördernd oder auf das zentrale Nervensystem wirkenden Wirkstoffe, einzeln oder in Kombination eingesetzt werden kann.

3. Verfahren zur Herstellung des zellulären hydrophilen Polyurethans gemäß einem der Ansprüche 1-2 durch Umsetzung der Monomere und Schäumungsmittel in Anwesenheit der gewünschten Wirkstoffe, **dadurch gekennzeichnet dass** mindestens 1 Wirkstoff bereits vor der Polymerisation in die Mischung der Monomere eingemischt wird.

4. Verwendung des zellulären hydrophilen Polyurethans gemäß einem der Ansprüche 1-2 zur Herstellung von medizinischem Gerät.

5. Verwendung des zellulären hydrophilen Polyurethans gemäß einem der Ansprüche 1-2 zur Herstellung einer mindestens einlagigen Wundauflage.

6. Verwendung des zellulären hydrophilen Polyurethans gemäß einem der Ansprüche 1-2 zur Herstellung einer Wundauflage für großflächige oder chronische Wunden.

7. Wundauflage enthaltend mindestens ein zelluläres hydrophiles Polyurethan gemäß einem der Ansprüche 1-2.

**Claims**

1. Cellular hydrophilic polyurethane gel containing polyhexamethylenebiguanide, PHMB and/or its hydrochloride as an antiseptic agent, **characterized in that** the antiseptic agent is present in the polyurethane gel as a microparticulate dispersion and/or homogeneous solution as well as a superabsorbent.

2. Cellular hydrophilic polyurethane according to claim 1, **characterized in that** at least one further therapeutic agent from the group of the broad-spectrum antibiotics, the antimicrobial actives, the antifungal actives, the antipathogenic peptides, the local anesthetics, the actives having an antiseptic, hemostatic, wound-healing, immunomodulatory or granulation-promoting effect or an effect on the central nervous system can additionally be used singly or in combination.

3. Process for producing the cellular hydrophilic polyurethane according to either of claims 1-2 by reaction of the monomers and foaming agent in the presence of the desired actives, **characterized in that** at least one active is mixed into the mixture of the monomers before polymerization.

4. Use of the cellular hydrophilic polyurethane according to either of claims 1-2 for producing medical equipment.

5. Use of the cellular hydrophilic polyurethane according to either of claims 1-2 for producing an at least one-ply wound contact material.

6. Use of the cellular hydrophilic polyurethane according to either of claims 1-2 for producing a wound contact material for large or chronic wounds.

7. Wound contact material comprising at least one cellular hydrophilic polyurethane according to either of claims 1-2.

**Revendications**

1. Gel cellulaire hydrophile de polyuréthanne, contenant du polyhexaméthylènebiguanide PHMB et/ou son chlorhydrate comme agent à action antiseptique, **caractérisé en ce que** l'agent à action antiseptique est présent dans le gel de polyuréthanne à l'état de répartition microparticulaire et/ou de solution homogène, à côté d'un hyperabsorbant.

2. Polyuréthanne cellulaire hydrophile suivant la revendication 1, **caractérisé en ce qu'**au moins un autre agent à action thérapeutique du groupe des antibiotiques à large spectre, des substances à activité antimicrobienne, des substances à activité antifongique, des peptides antipathogènes, des anesthésiques locaux, des substances actives douées d'activité antiseptique, hémostatique, cicatrisante, immunomodulatrice, facilitant la granulation et agissant sur le système nerveux central, peut être utilisé individuellement ou en combinaison.

3. Procédé de production du polyuréthanne cellulaire hydrophile suivant l'une des revendications 1 et 2 par réaction des monomères et d'agents moussants en présence des substances actives voulues, **caractérisé en ce qu'**au moins une substance active est déjà incorporée au mélange des monomères avant la polymérisation.

4.  Utilisation du polyuréthanne cellulaire hydrophile suivant l'une des revendications 1 et 2 pour la fabrication d'un appareil à usage médical.

5.  Utilisation du polyuréthanne cellulaire hydrophile suivant l'une des revendications 1 et 2 pour la production d'un revêtement pour plaies au moins monocouche.

6.  Utilisation du polyurétnanne cellulaire hydrophile suivant l'une des revendications 1 et 2 pour la production d'un revêtement pour plaies de grande étendue ou pour plaies chroniques.

7.  Revêtement pour plaies comprenant au moins un polyuréthanne cellulaire hydrophile suivant l'une des revendications 1 et 2.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente

- US 4479795 A
- US 5451424 A
- WO 9622114 A
- GB 2085454 A
- WO 03066116 A1
- US 20030149406 A1
- US 20040018227 A1
- EP 1175148 A1
- EP 0106439 A1
- GB 2290031 A1
- GB 2170713 A1
- WO 02100450 A1
- DD 139942
- WO 9940791 A1
- US 5869073 A1
- US 20040028722 A1
- WO 0203899 A1
- WO 9705910 A1
- US 4643181 A
- WO 8801877 A1
- DE OS2920501 A
- DE 3713601 A1
- WO 9407935 A1
- DE 3103499 A1
- DE 3103500 A1
- EP 0147588 A1

### In der Beschreibung aufgeführte Nicht-Patentliteratur

- Polyurethanes, Chemistry and Technology. High Polymers. Saunders-Frisch, Interscience Publishers, 1962, vol. XVI, 32-34
- Ullmann. vol. A3, 91-111
- ULLMANN. vol. A20, 461-479
- ULLMANN. vol. A23, 381-391
- Polyurethane. **BECKER/BRAUN.** Kunststoff-Handbuch. Carl Hanser Verlag, 1993, vol. 7, 115-118
- Polyurethane. **BECKER/BRAUN.** Kunststoff-Handbuch. Carl-Hauser Verlag München Wien, 1993, vol. 7, 139
- Absorbent Polymer Technology. Elsevier, 1990, 9-22
- Polyurethane. **BECKERBRAUN.** Kunststoff-Handbuch. Carl-Hauser Verlag München Wien, 1993, vol. 7, 139